# EUROPEAN PATENT APPLICATION

(11) **EP 1 166 782 A2**
(43) Date of publication of application: **02.01.2002**
(21) Application number: 00305468.1
(22) Date of filing: 29.06.2000
(51) Int. Cl.: A61K 31/216, A61P 31/14, A61P 31/18

(54) **Anti-retroviral pharmaceutical compositions**

(71) Applicant: Yang, Chi-Chiang, Taichung (TW); Tsai, Hsiu-Hsien, Ho-His Li, Ho-Mei Town, Chang-Hua Hsien (TW); Tsai, Shiou-Ren, Tsaotun Town, Nantou Hsien (TW)
(72) Inventor: Yang, Chi-Chiang, Taichung, Taiwan (TW)
(74) Representative: Woods, Geoffrey Corlett

(57) **Abstract**

The invention is directed to use of a composition in the manufacture of a medicament for the treatment or prophylaxis of a disorder related to retrovirus infection or for the inhibition of retrovirus replication, said composition comprising an effective amount of a compound of formula I 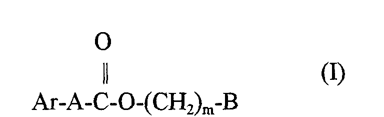 wherein
Ar represents aryl, which is unsubstituted or substituted with halo, hydroxyl or C₁₋₆-alkoxyl;
A represents C₁₋₆-alkylene, C₂₋₆-alkenylene or C₂₋₆-alkynylene;
M is an integer from 0 to 6; and
B represents hydrogen, C₁₋₆-alkyl or aryl, said aryl being unsubstituted or substituted with halo, hydroxyl, C₁₋₆-alkyl, C₂₋₆-alkenyl or C₂₋₆-alkynyl;
or a pharmaceutically acceptable salt thereof.

More particularly, the composition contains caffeic acid phenethyl ester (CAPE), methyl caffeate (MC), phenethyl dimethyl caffeate (PEDMC) or 4-bromocinnamic acid phenethyl ester (PBMC).

## Description

The invention is directed to a novel pharmaceutical composition comprising a compound of formula I. Said composition can be used in the treatment of disorders related to HIV and retrovirus and in the inhibition of HIV and retrovirus replication. In addition, said composition can optionally be combined with other anti-viral agents.

It is well known that human disorders of acquired immune deficiency syndrome (AIDS) are caused by human immunodeficiency virus (HIV).

HIV, as other viruses, cannot replicate itself if it does not occupy the biosynthesis apparatus of its infected host. The apparatus is enforced to produce structural proteins of viral offering. Such proteins are encoded by the genetic materials within the infected viral particles. However, as a retrovirus, the genetic material of HIV is RNA, rather than DNA as in the genome of the host cells. Accordingly, the viral RNA should be converted to DNA and then integrated into the genome of the host cells, so that the host cells can produce the desired viral proteins. The conversion of RNA to DNA is accomplished by the use of reverse transcriptase (RT), which is contained in the infected viral particles together with the RNA. Reverse transcriptase exhibits three known enzymatic functions, i.e., a RNA-dependent DNA polymerase, a ribonuclease and a DNA-dependent DNA polymerase. RT firstly plays as a RNA-dependent DNA polymerase to produce a single-stranded DNA copy of the viral RNA. And then, as a ribonuclease, RT releases the DNA produced from the original viral RNA and destroys the original RNA. Finally, as a DNA-dependent DNA polymerase, RT uses the first DNA strand as a template to produce the second complementary DNA strand. The two strands of the double-stranded DNA are then integrated into the genome of the host cells by another enzyme called integrase.

It is known that most compounds can inhibit the enzymatic function of HIV reverse transcriptase. One class of known HIV-1 RT inhibitors is nucleoside analogues. This class includes zidovudine (ZDV), 2',3'-dideoxyinosine (ddI) and 2',3'-dideoxycytidine (ddC). Another class is non-nucleoside analogues. Such class includes nevirapine, which is 11-cyclopropyl-5,11-dihydro-4-methyl-6H-dipyridino[3,2-b:2',3'-e][1,4]dia zepine-6-one. Non-nucleoside nevirapine and other especially suitable compounds are described in US patent No. 5,336,972.

However, since HIV can easily develop resistance to known anti-viral agents (*e.g.* protease inhibitors and reverse transcriptase inhibitors), the therapeutic effects of such agents are often lower than those expected. In addition, the dosages essential for such agents result in side effects for most patients. The most common disadvantage is the toxicity to normal cells. Therefore, persons skilled in medicine continuously develop new anti-viral agents and effective dosages thereof to obtain more effective treatments for disorders caused by HIV.

Recently, it has been found that the integrases essential for viral replication may be the target for developing another class of anti-viral agents. It is especially potential for use in developing anti-HIV inhibitors. In HIV and other retroviruses, the integration of a DNA copy obtained from the RNA genome into the host cells chromosome is essential for effective viral replication and multiplication of the viruses. More specifically, the enzymes are sequentially effected as follows: a dinucleotide unit is removed from the 3'-ends of the viral DNA (called "3'-processing"). The 3'-processed strands are transferred from the cytoplasm to the nucleus. The 3'-processed strands are incorporated into the corresponding host DNA in the nucleus through the offset cleavage of 5-base pair (called "strand displacement"). Moreover, integrase has not yet found the function similar to human cells. Therefore, integrase inhibitors may be useful for treating retroviral infections.

Despite the fact that integrases play an important role in the retroviral life cycle, the information concerning compounds with selective inhibition against HIV integrases is seldom reported. To date, the major classes of integrase inhibitors include DNA-binding agents, topoisomerase inhibitors, aurintricarboxylic acid, caffeic acid phenethyl ester (CAPE) and bis-catechols, and the like.

Many compounds are reported to inhibit HIV integrases in biochemical assays. However, most of these compounds possess little or no activities in tissue cultures and have no selectivity in their action mechanisms. These results show that the compounds do not have selectivity to eliminate the activation of HIV integrases or that the compounds inhibiting HIV integrase do not enter the cells. Particularly, although most compounds exhibit activity of inhibiting integrases in *in vitro* enzymatic assays, it is not yet demonstrated that such compounds have *in vivo* anti-HIV activity. For instance, both actinomycin D and CAPE have *in vitro* activity of inhibiting integrases. However, it is not reported that they have anti-HIV activity.

CAPE is the product of the propolis. It is known that CAPE has anti-mitogenic, anti-carcinogenic, anti-inflammatory and immunomodulatory properties. Moreover, CAPE can selectively inhibit virus-transformed and oncogene-transformed rodent cells and human tumor cells, including colon adenocarcinoma (HT-29), melanoma (HU-1, SK-MEL-28 and SK-MEL-MO), human breast carcinoma (MCF-7) and Fischer rat embryo fibroblast (CREF), and the like. CAPE can also cease the growth of human leukemia HL-60 cells and inhibit the synthesis of the DNA, RNA and proteins of HL-60 cells. The activation of NF-Kappa B by tumor necrosis factor can be dose- and time-dependently blocked by CAPE. CAPE can also be used as lipoxygenase inhibitor, performing anti-oxidation activity.

It is an object of the invention to provide a pharmaceutical composition for use in the treatment or prophylaxis of disorders related to HIV and retrovirus.

It is also an object of the invention to provide a pharmaceutical composition for inhibiting HIV and retrovirus viral replication.

It is a further object of the invention to provide a combination of the composition of the invention with known anti-viral agents.

### In the accompanying drawings:

Figure 1 represents the cellular activities in microculture tetrazolium tests for 24 hours.
Figure 2 represents the cellular activities in microculture tetrazolium tests for 48 hours.
Figure 3 represents the results of treating peripheral blood mononuclear cells infected with (A) macrophage-tropic (NL-43), (B) T cells-tropic (JRCSF) and (C) dual tropic (89.6) viruses, respectively, with caffeic acid phenethyl ester at different concentrations.
Figure 4 represents the results of treating peripheral blood mononuclear cells infected with (A) macrophage-tropic (NL-43), (B) T cells-tropic (JRCSF) and (C) dual tropic (89.6) viruses, respectively, with methyl caffeate at different concentrations.
Figure 5 represents the results of treating peripheral blood mononuclear cells infected with (A) macrophage-tropic (NL-43), (B) T cells-tropic (JRCSF) and (C) dual tropic (89.6) viruses, respectively, with phenethyl dimethyl caffeate at different concentrations.

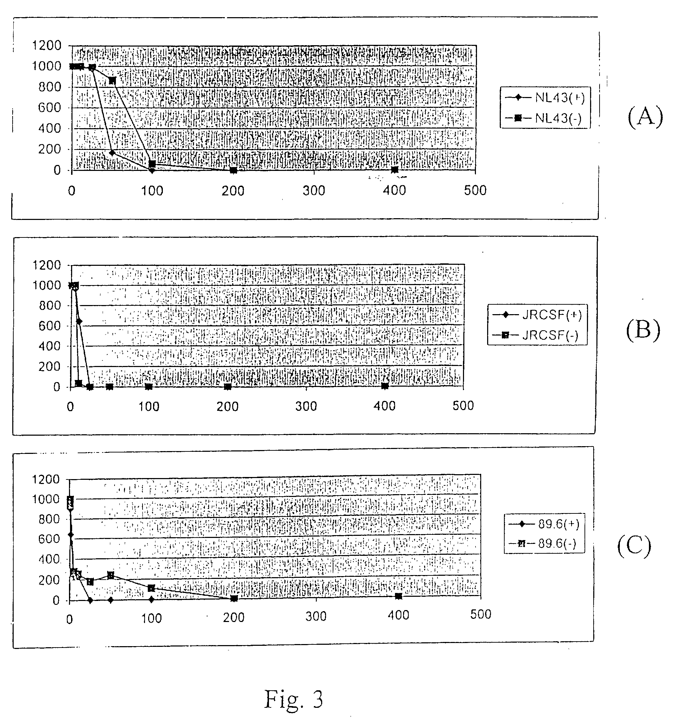

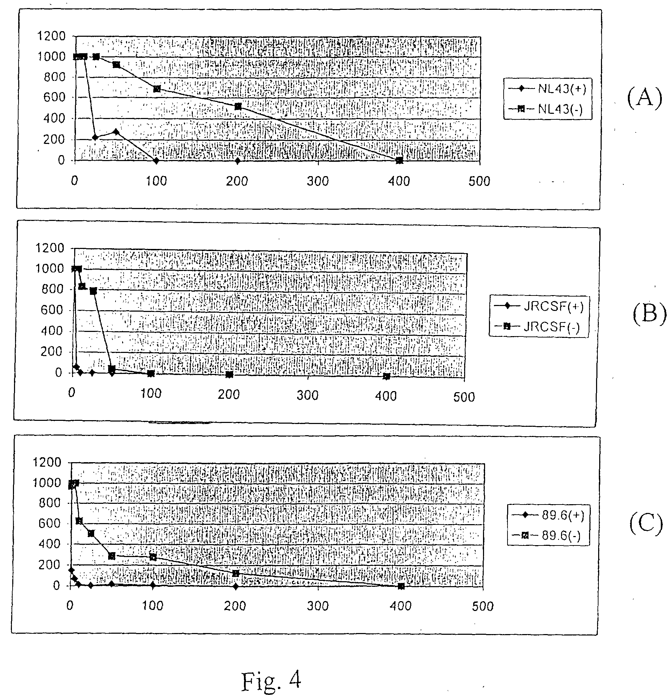

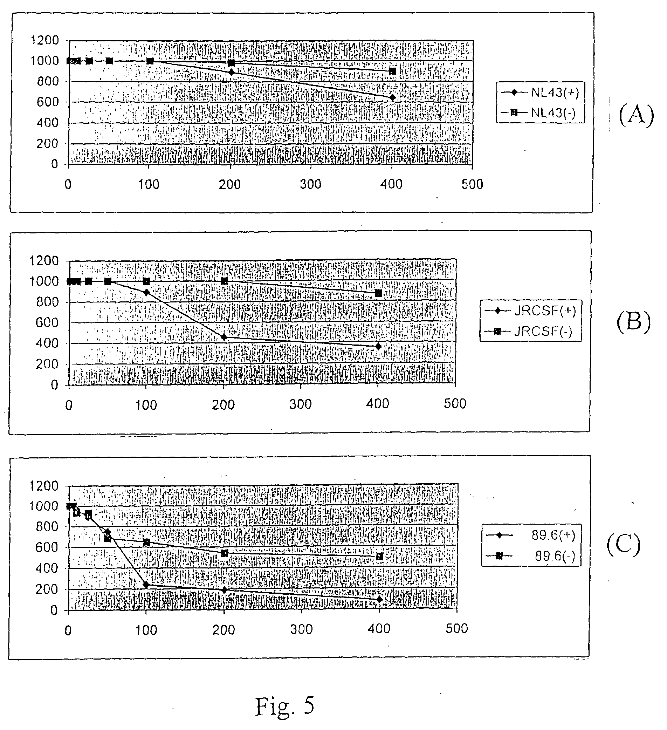

In Figures 3-5, (+) indicates maintaining the original concentration of the compound added after washing at the end of the infection.

In Figures 3-5, (-) indicates removing the compound after washing.

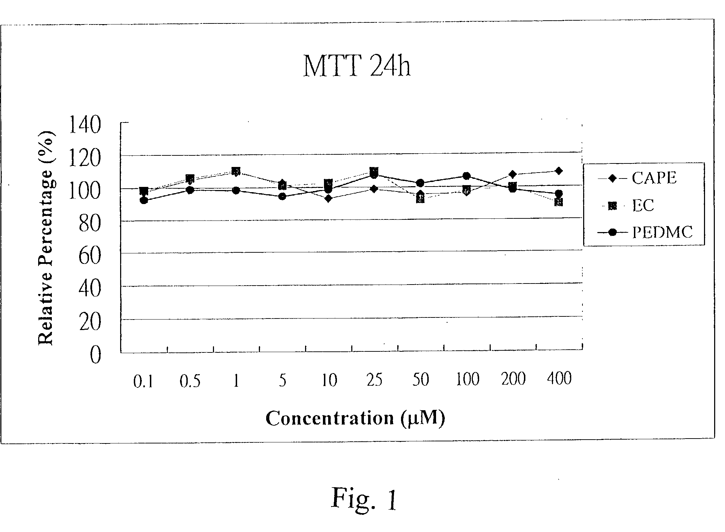

In Figures 1-5, the vertical axis represents the concentrations (µM)of the compounds tested.

In Figures 3-5, the horizontal axis represents the concentrations (unit) of the viral P24 antigen.

### In Figures 1-2, the horizontal axis represents relative percentage

The subject invention relates to the effects of the compounds of formula I and pharmaceutically acceptable salts thereof for use in the treatment or prophylaxis of disorders related to HIV and retrovirus. More specifically, the compounds of formula I can be used in the treatment or prophylaxis of disorders such as acquired immune deficiency syndrome (AIDS), AIDS-related complex (ARC) and adult T-cells leukemia. Therefore, the invention provides a pharmaceutical composition for the treatment or prophylaxis of disorders related to HIV and retrovirus, comprising an effective amount of a compound of formula I 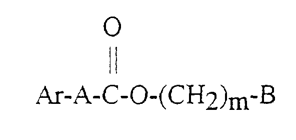 wherein
Ar represents aryl, which is unsubstituted or substituted with halo, hydroxyl or C₁₋₆-alkoxyl;
A represents C₁₋₆-alkylene, C₂₋₆-alkenylene or C₂₋₆-alkynylene;
m is an integer from 0 to 6; and
B represents hydrogen, C₁₋₆-alkyl or aryl, said aryl is unsubstituted or substituted with halo, hydroxyl, C₁₋₆-alkyl, C₂₋₆-alkenyl or C₂₋₆-alkynyl;
or pharmaceutically acceptable salts thereof, together with a pharmaceutically acceptable carrier.

Preferred compounds of formula I are those wherein
Ar represents phenyl or naphthyl, which is unsubstituted or substituted with halo, hydroxyl or C₁₋₆-alkoxyl;
A represents C₁₋₄-alkylene or C₂₋₄-alkenylene;
m is an integer from 0 to 3; and
B represents C₁₋₃-alkyl or phenyl, said phenyl is unsubstituted or substituted with halo, hydroxyl or C₁₋₆-alkyl;
or pharmaceutically acceptable salts thereof.

More preferred compounds of formula I are those wherein
Ar represents phenyl, which is substituted with halo, hydroxyl or methoxy;
A represents C₂₋₃-alkenylene;
m is an integer from 0 to 2; and
B represents methyl or phenyl;
or pharmaceutically acceptable salts thereof.

The most preferred compounds of formula I are selected from the group consisting of:
caffeic acid phenethyl ester (CAPE),
phenethyl dimethyl caffeate (PEDMC),
methyl caffeate (MC), and
4-bromocinnamic acid phenethyl ester,
or pharmaceutically acceptable salts thereof.

It was found that the compounds of formula I and pharmaceutically acceptable salts thereof have a substantial inhibiting activity in HIV replication. Further, the integrases of retrovirus and HIV are highly homologous. Accordingly, the invention also provides a pharmaceutical composition for inhibiting HIV and retrovirus replication, comprising a compound of formula I as defined above, together with a pharmaceutically acceptable carrier.

The compound of formula I may have one or more chiral centers. Therefore, it has various stereoisomer forms. That is, the compound of formula I includes all such isomers.

The compound of formula I and the starting materials for its preparation can be prepared by known processes, such as methods described in references (*e.g*., He zhao *et al*., *J. Med. Chem.* 1997, 40, 1186-1194; Chinthalapally B. Rao *et al*., *Chem. Biol. Interactions*, 84 (1992) 277-290; and the like). That is, the compounds of formula I can be prepared by reactions noted in such references under known and appropriate reaction disorders. The preparation can also be made by some of the known processes with minor modifications. However, such a preparation is not described in detail herein.

Nowadays, AIDS patients are treated with cocktail therapies. However, the HIV in some of the patients still developed resistance to known anti-viral agents. Effective treatments or prophylaxis therefore cannot be achieved. To achieve effective treatment or prophylaxis of AIDS or related disorders and inhibition of viral replications, it is necessary to use in combination with other anti-viral agents. Therefore, the invention provides a combination of a compound of formula I and one or more anti-viral agents. The anti-viral agents are selected from the group consisting of protease inhibitors such as indinavir, ritonavir or nelfinavir, nucleoside reverse transcriptase inhibitors such as zidovudine (ZDV), lamivudine (3TC), stavudine (d4T), 2',3'-dideoxyinosine (ddI) or 2',3'-dideoxycytidine (ddC), non-nucleoside reverse transcriptase inhibitors such as nevirapine, and integrase inhibitors.

It will be appreciated that the compounds of the combination may be administered simultaneously, either in the same or different pharmaceutical formulation or sequentially. If there is sequential administration, the delay in administering other active ingredients should not be at the expense of losing the benefit of a synergistic therapeutic effect of the combination of the active ingredients. It will also be understood that the various active ingredients used in the combination of the invention, or the physiologically functional derivatives of any thereof, whether presented simultaneously or sequentially, may be administered individually or in multiples or in any combination thereof. The active ingredients in the combinations are preferably administered simultaneously or sequentially in separate pharmaceutical formulations, most preferably simultaneously.

While it is possible for the active ingredients of the combination to be administered as the raw chemical it is preferable to present them as a pharmaceutical formulation. Pharmaceutical formulations according to the present invention comprise a combination according to the invention together with one or more pharmaceutically acceptable carriers or excipients and optionally other therapeutic agents. The carrier(s) must be acceptable in the sense of being compatible with the other ingredients of the formula and not deleterious to the recipient thereof. When the individual components of the combination are administered separately they are generally each presented as a pharmaceutical formulation.

It will be understood that the administration of the combination of the invention by means of a single patient pack, or patient packs of each formulation, containing within a package insert instructing the patient to the correct use of the invention is a desirable additional feature of this invention.

The compound of formula I and/or pharmaceutically acceptable salts thereof for use in the invention can form suitable dosage form in combination with at least one solid, liquid and/or semi-liquid excipient or adjuvant.

Useful excipients are inorganic or organic materials which are suitable for enterable (*e.g.*, oral), parenteral or topical administration, and are not interactive with the aforementioned compounds, *e.g.*, water, oils, benzyl alcohols, ethylene glycols, polyethylene glycols, gelatin, sugars such as lactose or starch, magnesium stearate, talc and petroleum jelly. In particular, tablets, pills, coated tablets, capsules, powders, particles, syrups or drops are for oral administrations; suppositories are for rectal administrations; solutions, as well as suspensions, emulsions, and injectable solutions are for parenteral administrations; and ointments, creams or powders are for topical administrations. The compounds of the invention can also be lyophilized and then used in the preparation of, for example, injectible solutions. These formulations can be sterilized, and/or contain adjuvants such as lubricants, preservatives, stabilizers and/or wetting agents, emulsifying agents, buffers, colorants, flavoring agents and/or sweetening agents.

Since the high dose administration of anti-viral agents to patients can easily result in toxicity, the invention provides a pharmaceutical composition or combination comprising safe and effective amount of a compound of formula I, wherein the safe and effective amount is from 0.1 to 1000 µM, preferably from 100 to 400 µM.

Specific dose level to be administrated to individual patient is determined by all possible factors, such as the activity of the specific compound employed, the age, body weight, general health, sex, diet, mode and time of administration, rate of excretion, drug combination, the severity of the disorder to be treated, and the like. Oral administration is the preferred route of administration.

### Example 1

### Synthesis of 4-Bromocinnamic Acid Phenethyl Ester

The solution of 500 mg (1 eq.) 4-bromobenzyladehyde and 562 mg (2 eq.) malonic acid in 4 ml of pyridine was mixed thoroughly, and 266 ml of piperidine was added. The mixture was heated to 80°C for 2 hours, and then heated to 115°C for 8 hours. After cooling, the reaction mixture was poured into 250 ml of cold water. The solution was acidified by slowly adding with 10 ml of HCl. Crystals were separated by filtration and then washed 4 times with cold water. The crude acid was dissolved in a solution of aqueous NaOH (1 : 20). The solution was filtered, diluted with 10 ml of cold water, and acidified with 1:1 HCl. The mixture was filtered, the crystals washed with 20 ml of cold water, and extracted with chloroform (Yield 77%).

4-bromocinnamic acid (1 eq.) was placed together with 8 ml of CHCl₃ and SO₂Cl₂ (3 eq.). The mixture was heated to 70°C for 7 hours and concentrated to give acid chloride. The acid chloride was then dissolved in CHCl₃ (10 ml) and the solution was added dropwise to the mixture of phenylethyl alcohol (2 eq.) and pyridine (2 eq.) in 10 ml of CHCl₃ over 5 miutes. The mixture was stirred for 30 minutes and purified with column chromatography to give 4-bromocinnamic acid phenethyl ester (Yield 88.6%).

### Example 2

Cytotoxicities of Caffeic Acid Phenethyl Ester (CAPE), Methyl Caffeate (MC) and Phenethyl Dimethyl Caffeate (PEDMC) to Peripheral Blood Mononuclear Cells (PBMC)

In 24-well plates, PBMC cells were exposed to various concentrations (0.1, 0.5, 1, 5, 10, 25, 50, 100, 200 and 400 µM) of CAPE, MC and PEDMC for 48 hours. Viability of the cells was assayed by the trypan blue dye exclusion method. More specifically, for determination of the growth inhibitory effect of MC, CAPE and PEDMC, cells were placed in 6-well dishes with several sub-cytotoxic concentrations of these agents for 48 hours. The concentration of DMSO was adjusted to be less than 0.5%. Cells from quadruplicated dishes were washed once with Hank's Balanced Salt Solution (HBSS). According to the process, the floating dead cells will be separated from the live monolayer cells. Then the cells were stained with trypan blue and counted. The total number of viable cells in the control group was considered as 100% viability and the agent-treated cells were compared with the control group for the determination of % viability. The % viability of agent-treated cells was calculated from the total number of viable cells in the control group and in the agent-treated group.

The results are shown in the following table. The viabilities of PBMC under high-dose treatments of CAPE, MC or PEDMC, even as high as 400 µM, have no substantial differences from those under low-dose treatments. (*e.g.*, 0.1-10µM). The results indicate that the compounds tested do not cause cytotoxicity to cells under such a high dose.

| Cytotoxicities of CAPE, MC and PEDMC to PBMC (the numbers of dead cells in 400 cells' count as follows and the numbers of dead cells in control being 9 cells) | | | | | | |
|---|---|---|---|---|---|---|
| concentration s (µM) | CAPE | | MC | | PEDMC | |
| | The numbers of dead cells in 400 cells' count | % viability | The numbers of dead cells in 400 cells' count | % viability | The numbers of dead cells in 400 cells' count | % viability |
| 400 | 8 | 392/391 (100.3%) | 7 | 393/391 (100.5%) | 6 | 394/391 (100.8%) |
| 200 | 7 | 393/391 (100.5%) | 8 | 392/391 (100.3%) | 7 | 393/391 (100.5%) |
| 100 | 11 | 389/391 (99.5%) | 7 | 393/391 (100.5%) | 10 | 390/391 (99.7%) |
| 50 | 12 | 388/391 (99.2%) | 11 | 389/391 (99.5%) | 10 | 390/391 (99.7%) |
| 25 | 5 | 395/391 (101%) | 11 | 389/391 (99.5%) | 8 | 392/391 (100.3%) |
| 10 | 10 | 390/391 (99.7%) | 7 | 393/391 (100.5%) | 9 | 391/391 (100%) |
| 5 | 10 | 390/391 (99.7%) | 9 | 391/391 (100%) | 11 | 389/391 (99.5%) |
| 1 | 7 | 393/391 (100.5%) | 12 | 388/391 (99.2%) | 7 | 393/391 (100.5%) |
| 0.5 | 9 | 391/391 (100%) | 7 | 393/391 (100.5%) | 10 | 390/391 (99.7%) |
| 0.1 | 11 | 389/391 (99.5%) | 9 | 391/391 (100%) | 11 | 389/391 (99.5%) |

### Example 3

### The Cellular Activities in Microculture Tetrazolium Tests

The cellular activities were tested according to method as described in Alley M.C. et al, Cancer Res. 1988; 48(3):589-601. The principle of the method resides in that live cells can reduce MTT., i.e., 3-[4,5-dimethylthiazol-2-yl]-2,5-diphenyltetrazolium bromide)(tetrazolium salt) to formazan crystal through the dehydrogenase metabolism in Mitochondria. The crystal was dissolved in propanol and OD values were determined at 570 nm. The OD values can indicate the numbers of the live cells. The growth conditions of the cells were observed firstly. The cells were diluted to 2x10⁴ cells/ml. 100µl sample taken by micropipet was placed in a 96-well plate. The plate was cultured for 24 hours as 37°C and 100µl of the compounds to be tested, which were prepared in medium, were added in each well. After culture for 48 hours, supernatant liquid was absorbed and 90 µl /well medium and 10µl /well MTT solution were added. After culture for 4 hours, supernatant liquid was absorbed and 100 µl DMSO and 10µl /well was added. Each well was homogeneously mixed. The absorbance was determined at 570 nm.

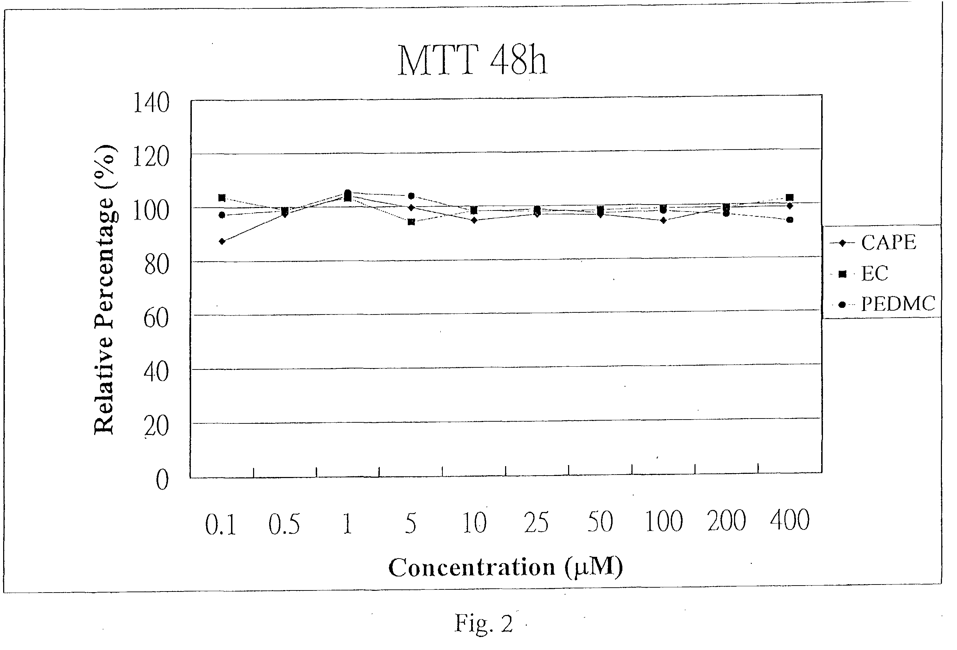

As showed in Figures 1 and 2, CAPE and EC there is no substantial effects between PEDMC, CAPE and EC. The results indicate the cells are actually alive.

### Example 4

### P24 Quantification for HIV and retrovirus-1

PBMCs were treated with various concentrations of CAPE, MC and PEDMC provided in Example 2 and simultaneously infected with macrophage-tropic (NL-43), T cells-tropic (JRCSF) and macrophage and T cells dual tropic (89.6) HIV-1 isolates. The next day, the added compounds were removed by washing methods or maintained at the same concentration. Samples were divided into two groups and the upernatants of the culture medium were collected at the 3 and 7 days after infection, respectively. The use of P24 EIA Reagent Kit of Abbott Laboratory compares the concentrations of viral P24 antigen with or without the compounds tested.

As showed in Figures 3-5, CAPE, MC and PEDMC have significantly inhibited viral replications. Especially, CAPE and MC at the concentration of 100 µM or less, can 100% inhibit viral replication of various HIV isolates. When the concentration of the compounds tested gradually decreases, the inhibition of viral replication gradually decreases. Moreover, the activities were not different due to various HIV isolates (NL-43, JRCSF or 89.6). It seemed that the occurrence of inhibition could not be achieved in the attachment stage but only after the subsequent viral penetration. Given the above, the results showed that CAPE, MC and PEDMC can inhibit replication of the HIV isolates with various tropism.
The detailed data in Figures 1-5 are as follows:

| Figure 1 | | | |
|---|---|---|---|
| MTT 24h | CAPE | EC | PEDMC |
| 0.1 | 97.49 | 98.38 | 92.38 |
| 0.5 | 104.67 | 105.82 | 98.72 |
| 1 | 109.25 | 110.22 | 98.26 |
| 5 | 102.41 | 101.18 | 94.33 |
| 10 | 93.07 | 102.41 | 98.72 |
| 25 | 98.65 | 109.46 | 107.27 |
| 50 | 95.49 | 92.15 | 102.03 |
| 100 | 96.15 | 98.07 | 106.06 |
| 200 | 106.8 | 99.69 | 97.93 |
| 400 | 108.62 | 89.69 | 94.8 |

| Figure 2 | | | |
|---|---|---|---|
| MTT 48h | CAPE | EC | PEDMC |
| 0.1 | 87.49 | 103.81 | 97.31 |
| 0.5 | 97.54 | 98.92 | 98.72 |
| 1 | 104.18 | 103.37 | 105.28 |
| 5 | 99.74 | 94.44 | 104.07 |
| 10 | 94.76 | 98.35 | 98.08 |
| 25 | 96.92 | 97.94 | 98.78 |
| 50 | 96.42 | 98.19 | 97.15 |
| 100 | 94.02 | 98.77 | 97.74 |
| 200 | 98.6 | 98.83 | 96.32 |
| 400 | 98.9 | 102.07 | 93.76 |

| Figure 3 | | | | | | | | |
|---|---|---|---|---|---|---|---|---|
| CAPE | NL43(+) | NL43(-) | CAPE | JRCSF(+) | JRCSF(-) | CAPE | 89.6(+) | 89.6(-) |
| 400 | 0 | 0 | 400 | 0 | 0 | 400 | 0 | 0 |
| 200 | 0 | 0 | 200 | 0 | 0 | 200 | 0 | 0 |
| 100 | 0 | 62.26 | 100 | 0 | 0 | 100 | 0 | 113.2 |
| 50 | 166.12 | 865.44 | 50 | 0 | 0 | 50 | 0 | 243.53 |
| 25 | 975.64 | 988.28 | 25 | 0 | 0 | 25 | 0 | 182.44 |
| 10 | 990.64 | 1000 | 10 | 642.72 | 34.42 | 10 | 230.32 | 255.21 |
| 5 | 1000 | 1000 | 5 | 965.62 | 1000 | 5 | 243.87 | 280.51 |
| 1 | 1000 | 1000 | 1 | 1000 | 1000 | 1 | 643.71 | 1000 |
| 0.5 | 1000 | 1000 | 0.5 | 1000 | 1000 | 0.5 | 899.22 | 949.75 |
| 0.1 | 1000 | 1000 | 0.1 | 1000 | 1000 | 0.1 | 1000 | 1000 |

| Figure 4 | | | | | | | | |
|---|---|---|---|---|---|---|---|---|
| MC | NL43(+) | NL43(-) | MC | JRCSF(+) | JRCSF(-) | MC | 89.6(+) | 89.5(-) |
| 400 | 0 | 2.53 | 400 | 0 | 0 | 400 | 0 | 0 |
| 200 | 0 | 518.45 | 200 | 0 | 0 | 200 | 0 | 124.97 |
| 100 | 0 | 687.24 | 100 | 0 | 0 | 100 | 8.91 | 278.43 |
| 50 | 273.07 | 922.63 | 50 | 0 | 42.64 | 50 | 18.4 | 286.46 |
| 25 | 220.63 | 1000 | 25 | 2.33 | 786.67 | 25 | 5.2 | 504.42 |
| 10 | 1000 | 1000 | 10 | 2.53 | 828.46 | 10 | 14.36 | 624.39 |
| 5 | 1000 | 1000 | 5 | 58.13 | 1000 | 5 | 66.78 | 1000 |
| 1 | 1000 | 1000 | 1 | 1000 | 1000 | 1 | 149.67 | 966.67 |
| 0.5 | 1000 | 1000 | 0.5 | 1000 | 1000 | 0.5 | 954.88 | 1000 |
| 0.1 | 1000 | 1000 | 0.1 | 1000 | 1000 | 0.1 | 1000 | 1000 |

| Figure 5 | | | | | | | | |
|---|---|---|---|---|---|---|---|---|
| PEDMC | NL43(+) | NL43(-) | PEDMC | JRCSF(+) | JRCSF(-) | PEDMC | 89.6(+) | 89.6(-) |
| 400 | 640.7 | 898.4 | 400 | 348.96 | 869.4 | 400 | 84.85 | 494.91 |
| 200 | 890.43 | 981.43 | 200 | 452.46 | 1000 | 200 | 187.59 | 539.82 |
| 100 | 1000 | 1000 | 100 | 892.45 | 1000 | 100 | 238.49 | 647 |
| 50 | 1000 | 1000 | 50 | 1000 | 1000 | 50 | 747.3 | 681.42 |
| 25 | 1000 | 1000 | 25 | 1000 | 1000 | 25 | 886.42 | 927.4 |
| 10 | 1000 | 1000 | 10 | 1000 | 1000 | 10 | 965.48 | 932.67 |
| 5 | 1000 | 1000 | 5 | 1000 | 1000 | 5 | 1000 | 1000 |
| 1 | 1000 | 1000 | 1 | 1000 | 1000 | 1 | 1000 | 1000 |
| 0.5 | 1000 | 1000 | 0.5 | 1000 | 1000 | 0.5 | 1000 | 1000 |
| 0.1 | 1000 | 1000 | 0.1 | 1000 | 1000 | 0.1 | 1000 | 1000 |

## Claims

1. Use of a composition comprising an effective amount of a compound of formula I 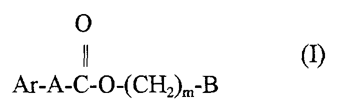 wherein,
Ar represents aryl, which is unsubstituted or substituted with halo, hydroxyl or C₁₋₆-alkoxyl;
A represents C₁₋₆-alkylene, C₂₋₆-alkenylene or C₂₋₆-alkynylene;
m is an integer from 0 to 6; and
B represents hydrogen, C₁₋₆-alkyl or aryl, said aryl being unsubstituted or substituted with halo, hydroxyl, C₁₋₆-alkyl, C₂₋₆-alkenyl or C₂₋₆-alkynyl;
or a pharmaceutically acceptable salt thereof in the manufacture of a medicament for use in a method of treatment or prophylaxis of a disorder related to retrovirus infection or for the inhibition of retrovirus replication.

2. Use according to claim 1, wherein the retrovirus is HIV.

3. Use according to claim 1 or 2 wherein,
Ar represents phenyl or naphthyl, which is unsubstituted or substituted with halo, hydroxyl or C₁₋₆-alkoxyl;
A represents C₁₋₄-alkylene or C₂₋₄-alkenylene;
m is an integer from 0 to 3; and
B represents C₁₋₃-alkyl or phenyl, said phenyl being unsubstituted or substituted with halo, hydroxyl or C₁₋₆-alkyl.

4. Use according to claim 3, wherein:
Ar represents phenyl, which is substituted with halo, hydroxyl or methoxy;
A represents C₂₋₃-alkenylene;
m is an integer from 0 to 2; and
B represents methyl or phenyl.

5. Use according to claim 1, wherein the compound of formula (I) is caffeic acid phenethyl ester (CAPE), phenethyl dimethyl caffeate (PEDMC), methyl caffeate (MC), or 4-bromocinnamic acid phenethyl ester.

6. Use according to any one of the preceding claims, wherein the disorder related to retrovirus infection is acquired immune deficiency syndrome (AIDS), AIDS-related complex (ARC) or adult T-cells leukemia.

7. Use according to any one of the preceding claims, wherein the effective amount of a compound of formula I is from 0.1 to 1000 µM.

8. Use according to claim 7, wherein the effective amount of a compound of formula I is from 100 to 400 µM.

9. A product containing:
(i) an effective amount of a composition as defined in any one of claims 1 to 8 or a pharmaceutically acceptable salt thereof; and
(ii) one or more antiviral agent,
for simultaneous, separate or sequential use in the treatment or prophylaxis of a disorder related to retrovirus infection or for the inhibition of retrovirus replication.

10. A product according to claim 9, wherein the retrovirus is HIV.

11. A product according to claim 9 or 10, wherein the antiviral agent(s) is /are a protease inhibitor, a nucleoside reverse transcriptase inhibitor, a non-nucleoside reverse transcriptase inhibitor or an integrase inhibitor.

12. A product according to claim 11, wherein the protease inhibitor is indinavir, ritonavir or nelfinavir.

13. Products according to claim 11, wherein the nucleoside reverse transcriptase inhibitor is zidovudine (ZDV), lamivudine (3TC), stavudine (d4T), 2',3'-dideoxyinosine (ddI) or 2',3'-dideoxycytidine (ddC).

14. A product according to claim 11, wherein the non-nucleoside reverse transcriptase inhibitor is nevirapine.

15. A pharmaceutical composition comprising a composition as defined in any one of claims 1 to 8 and a pharmaceutically acceptable carrier or diluent.

16. A pharmaceutical composition according to claim 15 further comprising one or more antiviral agent.

17. A composition as defined in any one of claims 1 to 8 for use in a method of treatment or prophylaxis of a disorder related to retrovirus infection or for the inhibition of retrovirus replication.
